# EUROPEAN PATENT SPECIFICATION

(11) **EP 2 817 051 B1**
(45) Date of publication and mention of the grant of the patent: **26.07.2017**
(21) Application number: 13751154.9
(22) Date of filing: 22.02.2013
(51) Int. Cl.: A61M 15/06, A24F 47/00

(54) **ELECTRONIC SMOKING ARTICLE**
ELEKTRONISCHER RAUCHARTIKEL
ARTICLE À FUMER ÉLECTRONIQUE

(30) Priority: 22.02.2012 US 201261601903 P
(43) Date of publication of application: 31.12.2014
(73) Proprietor: Altria Client Services LLC, Richmond, Virginia 23230 (US)
(72) Inventor: TUCKER, Christopher S., Midlothian, VA 23114 (US); KOBAL, Gerd, Sandy Hook, VA 23153 (US); JORDAN, Geoffrey Brandon, Midlothian, VA 23112 (US); KASOFF, Victor, Austin, TX 78701 (US)
(74) Representative: Carvajal y Urquijo, Isabel
(86) International application number: PCT/US2013/027424
(87) International publication number: WO 2013/126770

(56) References cited:
- WO-A1-2011/125058
- WO-A1-2011/125058
- CN-A- 102 166 044
- US-A- 4 735 217
- US-A- 4 945 931
- US-A- 4 993 436
- US-A- 5 144 962
- US-A- 5 894 841
- US-A- 6 155 268
- US-A1- 2003 108 342
- US-A1- 2006 196 518
- US-A1- 2010 031 968
- US-A1- 2011 315 152

## Description

### Working Environment

Many of the embodiments disclosed herein include electronic cigarettes and cigars which include heated capillary aerosol generators and manually operative arrangements to deliver liquid from a liquid supply source to the capillary while the capillary is being heated. The heated capillary volatilizes a liquid such as by way of the teachings set forth in US patent 5, 743, 251.

The present invention is defined in the apparatus claim 1 and in the method claim 23 with preferred embodiments according to the claims dependent thereon.

A device according to the first part of claims 1 and 23 is known from US 2006/0196518 A1.

### Summary of Selected Features

An electronic cigarette comprises an outer cylindrical housing extending in a longitudinal direction, a liquid supply formed of an elastomeric material and containing a liquid material, a capillary tube having an inlet and an outlet, the inlet in communication with the outlet of the liquid supply, a power supply operable to apply voltage across a heater operable to heat the capillary tube to a temperature sufficient to at least initially volatilize liquid material contained within the capillary tube, a mixing chamber downstream of the capillary tube and at least one air inlet operable to deliver air drawn into the mixing chamber. The liquid supply is at least partially contained within the outer cylindrical housing and includes an outlet. The liquid supply is adapted to be compressed so as to pump liquid material from the liquid supply and through the outlet. The heater is adapted to be activated when the liquid supply is compressed so as to heat the capillary tube. Air mixed with the volatilized liquid material in the mixing chamber forms an aerosol

The electronic cigarette can also include a mouth-end insert having at least one outlet. The mouth-end insert is in fluid communication with the mixing chamber so as to deliver aerosol to a smoker.

### Brief Description of the Drawings

Figure 1 is a cross-sectional view of an electronic cigarette according to a first embodiment;
Figure 2 is a perspective view of the electronic cigarette according to a second embodiment.
Figure 3 is an exploded view of the electronic cigarette of Figure 2.
Figure 4 is an enlarged view, top view of a fitting operable to hold a liquid supply containing liquid within the electronic cigarette of Figures 2 and 3.
Figure 5 is a cross-sectional view of the electronic cigarette of Figure 2.
Figure 6 is a cross-sectional view of an electronic cigarette according to a third embodiment.
Figure 7 is a perspective view of the electronic cigarette of Figure 2 including a liquid supply.

### Detailed Description

An electronic cigarette provides a flexible and/or compressible liquid supply, which is squeezed by a smoker to simultaneously pump liquid from the liquid supply to a capillary tube and activate a heater. Optionally, the electronic cigarette can include a check valve to limit the amount of liquid that can be pumped with each compression of the liquid supply and/or to prevent drawback of air into the liquid supply. Thus, the electronic cigarette is manually controlled and does not need an electromechanical pump, thereby extending battery life. Moreover, the use of a manual pump and capillary tube removes the need for a wick or other fibrous material in the electronic cigarette which may become entrained in the air path. In addition, a manual pump allows for the supply of liquid to the capillary tube for as long as the smoker applies pressure to the liquid supply. Thus, the continuity of the sensorial experience is maintained because the smoker is supplied with the same flavor from start to finish based on smoker preference. Moreover, the use of a capillary tube in an electronic cigarette allows for positioning of air inlets downstream of the heater so as to reduce temperature fluctuations at the heater. Finally, the electronic cigarette provides a sealed liquid supply that protects the liquid formulation contained therein from the atmosphere until use so as to avoid evaporation and/or degradation.

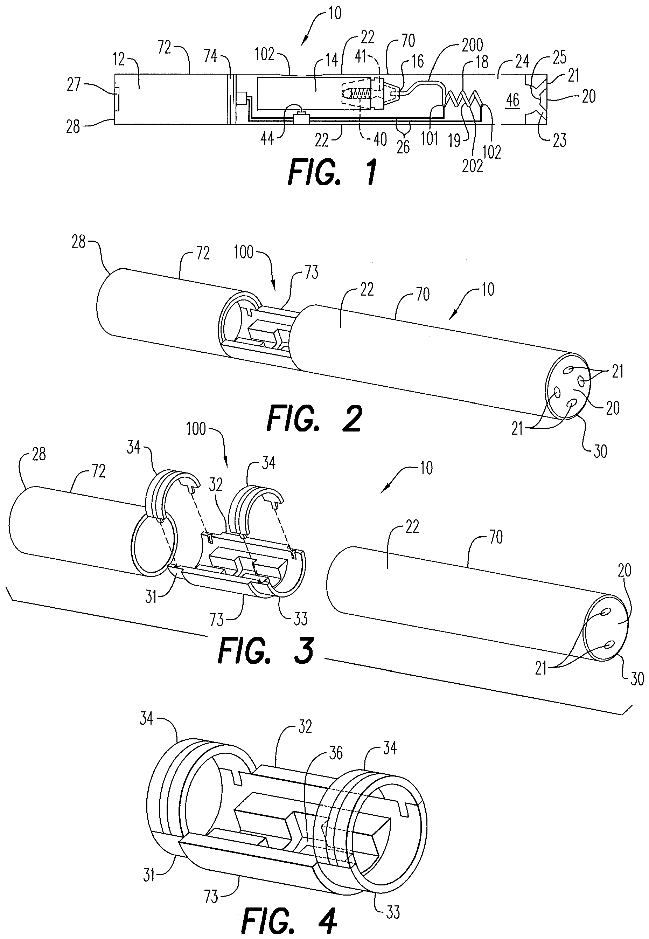

As shown in Figure 1, an electronic cigarette 10 comprises a replaceable cartridge (or first section) 70 and a reusable fixture (or second section) 72, which are coupled together at a threaded joint 74 or by other convenience such as a snug-fit, snap-fit, detent, clamp and/or clasp. The first section 70 can house a mouth-end insert 20, a capillary tube 18, a heater 19 to heat at least a portion of the capillary tube 18 (which may comprise a heatable portion 19 of the capillary tube 18 itself) and a liquid supply 14. The second section 72 can house a power supply 12 and control circuitry. The threaded portion 74 of the section 72 can be connected to a battery charger when not connected to the first section 70 for use so as to charge the battery.

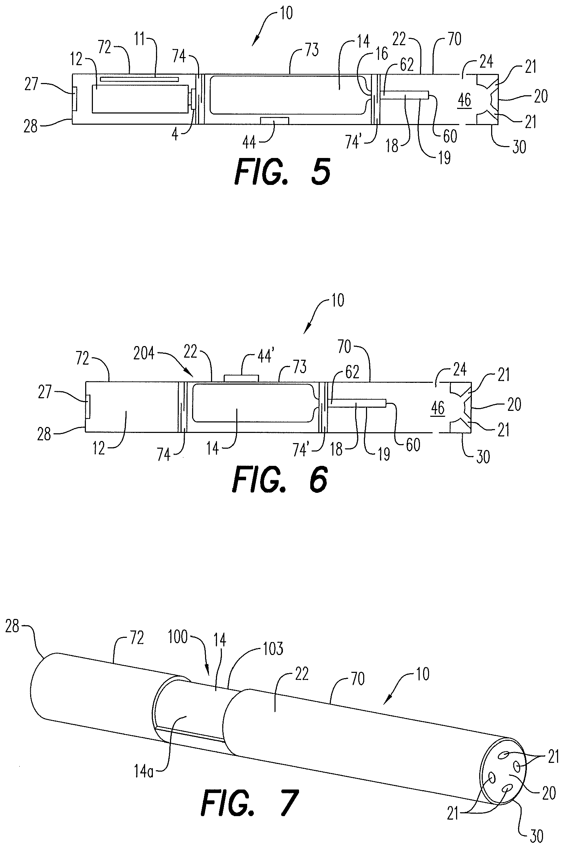

In an alternative embodiment, as shown in Figures 2, 3, 5, 6 and 7, the electronic cigarette 10 can also include a middle section (third section) 73, which can house only the liquid supply 14. The middle section 73 can be adapted to be fitted with a threaded joint 74' at an upstream end of the first section 70 and a threaded joint 74 at a downstream end of the second section 72, as shown in Figures 5 and 6. In this embodiment, the first section 70 houses the heated capillary tube 18 and mouth-end insert 20, while the second section 72 houses the power supply 12.

Preferably, the first section 70, second section 72 and optional third section 73 include an outer cylindrical housing 22 extending in a longitudinal direction along the length of the electronic cigarette 10. Preferably, the outer cylindrical housing 22 is elastomeric so as to be flexible and/or compressible such that the smoker can apply pressure and/or squeeze the liquid supply 14 to pump liquid to the capillary tube 18 and activate the heater.

As shown in Figures 2, 3 and 7, the outer cylindrical housing 22 can include a cutout 100 which allows a smoker to directly contact the liquid supply 14. Thus, the liquid supply 14 is designed to be part of the outer cylindrical housing 22 so that the outer cylindrical housing 22 is substantially continuous along the length thereof. A wall 14a of the liquid supply 14 can form a portion of the outer cylindrical housing 22 of the electronic cigarette. Preferably, the electronic cigarette is formed so that the diameter of the electronic cigarette is substantially uniform along the length thereof. When the liquid supply 14 forms a portion of the outer cylindrical housing 22, the remainder of the outer cylindrical housing 22 can be substantially rigid or elastomeric.

Alternatively, as shown in Figure 6, the outer cylindrical housing 22 is substantially continuous along the length thereof and can be rigid. A pressure activated switch 44' can be positioned on an outer surface of the outer cylindrical housing 22, which acts to apply pressure to the liquid supply 14 and simultaneously activates the heater. In this embodiment, the liquid supply 14 is formed of an elastomeric material so that upon application of manual pressure to the pressure switch, pressure is also applied to a side of the liquid supply 14 so as to force liquid through the outlet 16 of the liquid supply 14 to the capillary tube 18. By applying manual pressure to the pressure switch, the power supply is activated and an electric current heats the liquid in the capillary tube 18 via electrical contacts so as to volatilize the liquid.

As shown in Figure 1, in another embodiment, the outer cylindrical housing 22 can be flexible along the length thereof and fully cover the liquid supply 14. In use, a smoker can apply pressure to the outer cylindrical housing 22 adjacent the liquid supply 14 so as to pump the liquid and simultaneously apply pressure to a pressure switch, which activates the control circuitry and causes the power supply to send an electric current to the heat the heater. In one embodiment, a depression 102 can be formed in the outer cylindrical housing 22 to indicate where the smoker should apply pressure. The depression 102 can extend fully or partially about the circumference of the outer cylindrical housing 22.

In one embodiment, the middle section 73 is disposable and the first section 70 and/or second section 72 is reusable. In another embodiment, the first section 70 can also be replaceable so as to avoid the need for cleaning the capillary tube 18. The sections 70, 72, 73 can be attached by a threaded connection whereby the middle section 73 can be replaced when the liquid supply 14 is used up.

In the preferred embodiment, the liquid supply 14 is a tubular, elongate body formed of an elastomeric material so as to be flexible and/or compressible when squeezed. Preferably, the elastomeric material can be selected from the group consisting of silicone, plastic, rubber, latex, and combinations thereof.

Preferably, the compressible liquid supply 14 has an outlet 16 which is in fluid communication with a capillary tube 18 so that when squeezed, the liquid supply 14 can deliver a volume of liquid material to the capillary tube 18. Simultaneous to delivering liquid to the capillary, the power supply 12 is activated upon application of manual pressure to the pressure switch and the capillary tube 18 is heated to form a heated section wherein the liquid material is volatilized. Upon discharge from the heated capillary tube 18, the volatilized material expands, mixes with air and forms an aerosol.

Preferably, the liquid supply 14 extends longitudinally within the outer cylindrical housing 22 of the first section 70 (shown in Figure 1) or the middle section 73 (shown in Figure 5). Moreover, the liquid supply 14 comprises a liquid material which is volatilized when heated and forms an aerosol when discharged from the capillary tube 18.

In the preferred embodiment, the capillary tube 18 includes an inlet end 62 in fluid communication with the outlet 16 of the liquid supply 14, and an outlet end 60 (shown in Figures 5 and 6) operable to expel volatilized liquid material from the capillary tube 18.

Preferably, the capillary tube 18 has an internal diameter of 0.01 to 10 mm, preferably 0.05 to 1 mm, and more preferably 0.05 to 0.4 mm. For example, the capillary tube can have an internal diameter of about 0.05 mm. Capillary tubes of smaller diameter provide more efficient heat transfer to the fluid because, with the shorter the distance to the center of the fluid, less energy and time is required to vaporize the liquid. Alternatively, the capillary tube has an internal cross sectional area of 8 x 10⁻⁵ to 80 mm², preferably 0.002 to 0.8 mm², more preferably 0.002 to 0.05 mm². For example, the capillary tube can have an internal cross sectional area of about 0.002 mm².

Also preferably, the capillary tube 18 may have a length of about 5 mm to about 72 mm, more preferably about 10 mm to about 60 mm or about 20 mm to about 50 mm. For example, the capillary tube 18 can be about 50 mm in length and arranged such that a downstream, about 40 mm long portion of the capillary tube 18 forms a heated section 202 and an upstream, about 10 mm long portion 200 of the capillary tube 18 remains relatively unheated when the heater 19 is activated (shown in Figure 1).

In one embodiment, the capillary tube 18 is substantially straight. In other embodiments, the capillary tube 18 is coiled and/or includes one or more bends therein to conserve space.

In the preferred embodiment, the capillary tube 18 is formed of a conductive material, and thus acts as its own heater 19 by passing current through the tube. The capillary tube 18 may be any electrically conductive material capable of being resistively heated, while retaining the necessary structural integrity at the operating temperatures experienced by the capillary tube 18, and which is nonreactive with the liquid material. Suitable materials for forming the capillary tube 18 are selected from the group consisting of stainless steel, copper, copper alloys, porous ceramic materials coated with film resistive material, Inconel^{®} available from Special Metals Corporation, which is a nickel-chromium alloy, Nichrome^{®}, which is also a nickel-chromium alloy, and combinations thereof.

In one embodiment, the capillary tube 18 is a stainless steel capillary tube 18, which serves as a heater 19 via electrical leads 26 attached thereto for passage of direct or alternating current along a length of the capillary tube 18. Thus, the stainless steel capillary tube 18 is heated by resistance heating. The stainless steel capillary tube 18 is preferably circular in cross section. The capillary tube 18 may be of tubing suitable for use as a hypodermic needle of various gauges. For example, the capillary tube 18 may comprise a 32 gauge needle has an internal diameter of 0.11 mm and a 26 gauge needle has an internal diameter of 0.26 mm.

In another embodiment, the capillary tube 18 may be a non-metallic tube such as, for example, a glass tube. In such an embodiment, the heater 19 is formed of a conductive material capable of being resistively heated, such as, for example, stainless steel, Nichrome^{®} or platinum wire, arranged along the glass tube. When the heater arranged along the glass tube is heated, liquid material in the capillary tube 18 is heated to a temperature sufficient to at least partially volatilize liquid material in the capillary tube 18.

Preferably, at least two electrical leads 26 are bonded to a metallic capillary tube 18. In the preferred embodiment, the at least two electrical leads 26 are brazed to the capillary tube 18. Preferably, one electrical lead 26 is brazed to a first, upstream portion 101 of the capillary tube 18 and a second electrical lead 26 is brazed to a downstream, end portion 102 of the capillary tube 18, as shown in Figure 1.

In use, once the capillary tube 18 is heated, the liquid material contained within a heated portion of the capillary tube 18 is volatilized and ejected out of the outlet 60 (shown in Figures 5 and 6) where it expands and mixes with air and forms an aerosol in a mixing chamber 46.

Preferably, the electronic cigarette 10 also includes at least one air inlet 24 operable to deliver air to the mixing chamber 46. Preferably, the air inlets 24 to the mixing chamber 46 are arranged downstream of the capillary tube 18 so as to minimize drawing air along the capillary tube and thereby avoid cooling of the capillary tube 18 during heating cycles. In use, the volatilized material expands out of the capillary tube 18 and into the mixing chamber 46 where it can mix with air to form an aerosol which is then drawn through the mouth-end insert 20. In the preferred embodiment, the at least one air inlet 24 includes one or two air inlets. Alternatively, there may be three, four, five or more air inlets. Altering the size and number of air inlets 24 can also aid in establishing the resistance to draw of the electronic cigarette 10.

Preferably, the capillary tube 18 is spaced sufficiently apart from the mouth-end of the electronic cigarette 10 to protect it and a smoker's fingers from each other should the mouth-end insert 20 be removed.

In the preferred embodiment, the liquid supply 14 may include a check valve 40, shown in Figure 1. The check valve 40 is operable to maintain the liquid material within the liquid supply, but opens when the liquid supply 14 is squeezed and pressure is applied. Preferably, the check valve 40 opens when a critical, minimum pressure is reached so as to avoid inadvertent dispensing of liquid material from the liquid supply 14 or activating the heater 19. Preferably, the critical pressure needed to open the check valve 40 is essentially equal to or slightly less than the pressure required to press a pressure switch 44 to activate the heater 19. Preferably, the pressure required to press the pressure switch 44 is high enough such that accidental heating is avoided. Such arrangement avoids activation of the heater 19 in the absence of liquid being pumped through the capillary.

Advantageously, the use of a check valve 40 also aids in limitaing the amount of liquid that is drawn back from the capillary upon release of pressure upon the liquid supply 14 (and/or the switch 44). Withdrawal of liquid from the capillary at conclusion of a puff (or activation) is desirous. The presence of residual liquid in the capillary at the initiation of a new puff cycle can lead to undesirable sputtering of liquid from the heated capillary at the beginning of activation. Withdrawing the liquid via "drawback" as a result of the supply bladder 14 returning to toward its original, uncompressed state can avoid such sputtering, but can, if left unchecked, lead to air being drawn into the liquid supply bladder 14. Presence of air degrades pumping performance of the supply bladder. Use of a check valve 40 can be configured to allow a desired, limited amount of drawback to occur, such that drawback of liquid occurs without air being not drawn into the supply bladder 14. Such arrangement may be achieved by adjusting the size or the closing action of the check valve shown in Figure 1.

Once pressure upon the liquid supply 14 is relieved, the check valve 40 closes. The heated capillary tube 18 discharges liquid remaining downstream of the check valve 40. Advantageously, the capillary tube 18 is purged once a smoker has stopped compressing the liquid supply 14 because any liquid remaining in the tube is expelled during heating.

The check valve is a one-way or non-return valve, which allows the liquid to flow in a single direction so as to prevent backflow or liquid and air bubbles in the liquid supply. The check valve can be a ball check valve, a diaphragm check valve, a swing check valve, a stop-check valve, a lift-check valve, an in-line check valve or a duckbill valve. To assure purging, the heating cycle may be extended by a controlled amount beyond release of pressure on the switch 44 and/or closure of the check valve 40.

Optionally, a critical flow orifice 41 is located downstream of the check valve 40 to establish a maximum flow rate of liquid to the capillary tube 18.

Adjacent the liquid supply 14 is the pressure switch 44. The pressure switch 44 is positioned such that when the liquid supply 14 is squeezed, the pressure switch 44 communicates with the control circuitry to supply power and activate the heater 19 which in turn heats the capillary tube 18 to volatilize the liquid material therein.

In one embodiment, as shown in Figure 6, the pressure switch 44' can be located on an outer surface 204 of the electronic cigarette 10 and the pressure switch 44' is pressed to activate the heater 19 and squeeze the liquid supply 14. The control circuitry is integrated with the pressure switch 44 and supplies power to the heater 19 responsive to pressing the pressure switch. Preferably, the pressure switch 44, 44' is adjacent the liquid supply 14 so that a single action is needed to simultaneously activate the heater 19 and supply liquid to the capillary tube 18.

As shown in Figures 3 and 4, the liquid 14 can be held within a fitting 32. The fitting 32 can include a recess 36 into which the pressure switch 44 is recessed. Clamps 34 hold the liquid supply 14 within the fitting 32. Each end 31, 33 of the fitting 32 can be threaded or otherwise configured to mate with the first section 70 and the second section 72 of the electronic cigarette 10. When the fitting 32 is used, the liquid supply 14 can be configured to be removable and replaceable once the liquid supply is used. Thus, a new liquid supply 14 could be secured within the fitting 32 for continued smoking.

In the preferred embodiment, the power supply 12 includes a battery arranged in the electronic cigarette 10 such that the anode is downstream of the cathode. A battery anode connector 4 (shown in Figure 5) contacts the downstream end of the battery. The heater 19 can be connected to the battery by two spaced apart electrical leads 26 (also shown in Figure 1). The power supply 12 is operable to apply voltage across the heater 19 associated with the capillary tube 18 and volatilize liquid material contained therein according to a power cycle of either a predetermined time period, such as a 5 second period, or for so long as pressure is applied to the liquid supply 14 and/or the pressure activated switch 44.

Preferably, the electrical contacts or connection between the heater 19 and the electrical leads 26 are highly conductive and temperature resistant while the heatable portion 19 of the capillary tube 18 is highly resistive so that heat generation occurs primarily along the heater 19 and not at the contacts.

The battery can be a Lithium-ion battery or one of its variants, for example a Lithium-ion polymer battery. Alternatively, the battery may be a Nickel-metal hydride battery, a Nickel cadmium battery, a Lithium-manganese battery, a Lithium-cobalt battery or a fuel cell. In that case, preferably, the electronic cigarette 10 is usable by a smoker until the energy in the power supply is depleted. Alternatively, the power supply 12 may be rechargeable and include circuitry allowing the battery to be chargeable by an external charging device. In that case, preferably the circuitry, when charged, provides power for a pre-determined number of puffs, after which the circuitry must be re-connected to an external charging device.

Preferably, the electronic cigarette 10 also includes control circuitry which can be on a printed circuit board 11. Once the pressure switch is pressed, the power supply is activated and supplies power to the heater 19. The control circuitry 11 can also include a heater activation light 27 operable to glow when the heater 19 is activated. Preferably, the heater activation light 27 comprises an LED and is at an upstream end 28 of the electronic cigarette 10 so that the heater activation light 27 takes on the appearance of a burning coal during a puff. Moreover, the heater activation light 27 can be arranged to be visible to the smoker. In addition, the heater activation light 27 can be utilized for cigarette system diagnostics. The light 27 can also be configured such that the smoker can activate and/or deactivate the light 27 when desired, such that the light 27 would not activate during smoking if desired.

The control circuitry 11 is integrated with the pressure switch 44 and supplies power to the heater 19 of the capillary tube 18 responsive to pressing the pressure switch 44, preferably with a maximum, time-period limiter (e.g. a timing circuit). The control circuitry 11 also includes a timer operable to limit the time for which power is supplied to the heater 19.

The time-period of the electric current supply to the heater 19 may be preset depending on the amount of liquid desired to be vaporized. The control circuitry 11 can be programmable for this purpose. The control circuitry can be an application specific integrated circuit (ASIC).

Preferably, when activated, the heater 19 heats a portion of the capillary tube 18 for less than about 10 seconds, more preferably less than about 7 seconds. Thus, the power cycle (or maximum puff length) can range in period from about 2 seconds to about 10 seconds (e.g., about 3 seconds to about 9 seconds, about 4 seconds to about 8 seconds or about 5 seconds to about 7 seconds).

In the preferred embodiment, the liquid supply 14 includes a liquid material which has a boiling point suitable for use in the electronic cigarette 10. If the boiling point is too high, the heater 19 will not be able to vaporize liquid in the capillary tube 18. However, if the boiling point is too low, the liquid may vaporize without the heater 19 being activated.

Preferably, the liquid material includes a tobacco-containing material including volatile tobacco flavor compounds which are released from the liquid upon heating. The liquid may also be a tobacco flavor containing material and/or a nicotine-containing material. Alternatively, or in addition, the liquid may include a non-tobacco material and/or may be nicotine-free. For example, the liquid may include water, solvents, ethanol, plant extracts and natural or artificial flavors. Preferably, the liquid further includes an aerosol former. Examples of suitable aerosol formers are glycerine and propylene glycol.

In use, liquid material is transferred from the liquid supply 14 to the heated capillary tube 18 by manual pumping caused by squeezing of the liquid supply 14.

As shown in Figures 1, 5 and 6 the electronic cigarette 10 further includes a mouth-end insert 20 having at least two off-axis, preferably diverging outlets 21. Preferably, the mouth-end insert 20 is in fluid communication with the mixing chamber 46 and includes at least two diverging outlets 21. (e.g, 3, 4, 5, or preferably 6 to 8 outlets or more). Preferably, the outlets 21 of the mouth-end insert 20 are located at ends of off-axis passages 23 and are angled outwardly in relation to the longitudinal direction of the electronic cigarette 10 (i.e., divergently). As used herein, the term "off-axis" denotes at an angle to the longitudinal direction of the electronic cigarette. Also preferably, the mouth-end insert (or flow guide) 20 includes outlets uniformly distributed around the mouth-end insert 20 so as to substantially uniformly distribute aerosol in a smoker's mouth during use. Thus, as the aerosol passes into a smoker's mouth, the aerosol enters the mouth and moves in different directions so as to provide a full mouth feel as compared to electronic cigarettes having an on-axis single orifice which directs the aerosol to a single location in a smoker's mouth.

In addition, the outlets 21 and off-axis passages 23 are arranged such that droplets of unaerosolized liquid material carried in the aerosol impact interior surfaces 25 of the mouth-end insert 20 and/or interior surfaces of the off-axis passages 23 such that the droplets are removed or broken apart. In the preferred embodiment, the outlets 21 of the mouth-end insert 20 are located at the ends of the off-axis passages 23 and are angled at 5 to 60° with respect to the central longitudinal axis of the electronic cigarette 10 so as to more completely distribute aerosol throughout a mouth of a smoker during use and to remove droplets.

Preferably, each outlet 21 has a diameter of about 0.015 inch to about 0.090 inch (e.g., about 0.020 inch to about 0.040 inch or about 0.028 inch to about 0.038 inch). The size of the outlets 21 and off-axis passages 23 along with the number of outlets 21 can be selected to adjust the resistance to draw (RTD) of the electronic cigarette 10, if desired.

As shown in Figure 1, an interior surface 25 of the mouth-end insert 20 can comprise a generally domed surface. Alternatively, the interior surface 25 of the mouth-end insert 20 can be generally cylindrical or frustoconical, with a planar end surface. Preferably, the interior surface is substantially uniform over the surface thereof or symmetrical about the longitudinal axis of the mouth-end insert 20. However, in other embodiments, the interior surface can be irregular and/or have other shapes.

Preferably, the mouth-end insert 20 is affixed within the outer cylindrical housing 22 of the cartridge 72.

In a preferred embodiment, the electronic cigarette 10 is about the same size as a conventional cigarette. In some embodiments, the electronic cigarette 60 can be about 80 mm to about 110 mm long, preferably about 80 mm to about 100 mm long and about 7 mm to about 8 mm in diameter. For example, in an embodiment, the electronic cigarette is about 84 mm long and has a diameter of about 7.8 mm.

The outer cylindrical housing 22 of the electronic cigarette 10 may be formed of any suitable material or combination of materials. Examples of suitable materials include metals, alloys, plastics or composite materials containing one or more of those materials, or thermoplastics that are suitable for food or pharmaceutical applications, for example polypropylene, polyetheretherlcetone (PEEK), ceramic, low density polyethylene (LDPE) and high density polyethylene (HDPE). Preferably, the material is light and non-brittle. More preferably, at least a portion of the outer cylindrical housing 22 is elastomeric so as to allow a smoker to squeeze the liquid supply 14 during smoking to release liquid material therefrom and activate the heater 19. Thus, the outer cylindrical housing 22 can be formed of a variety of materials including plastics, rubber and combinations thereof. In a preferred embodiment, the outer cylindrical housing 22 is formed of silicone. The outer cylindrical housing 22 can be any suitable color and/or can include graphics or other indicia printed thereon.

In an embodiment, the volatilized material formed as described herein can at least partially condense to form an aerosol including particles. Preferably, the particles contained in the vapor and/or aerosol range in size from about 0.5 micron to about 4 microns, preferably about 1 micron to about 4 microns. In the preferred embodiment, the vapor and/or aerosol has particles of about 3.3 microns or less, more preferably about 2 nanometers (nm) or less. Also preferably, the particles are substantially uniform throughout the vapor and/or aerosol.

In another embodiment, in lieu of a pressure switch, a flow sensor could be arranged to detect flow being pumped to the capillary, and serve as the switch between the power source 12 and heater 19. Furthermore, a puff sensor could be added and coupled with the flow sensor such that signals from both, indicative of both liquid flow and a puff, would connect the battery to the heater 19.

The teachings herein are applicable to electronic cigars, and references to "electronic cigarette(s)" is intended to be inclusive of electronic cigars and the like. Moreover, references to "electronic smoking articles" is intended to be inclusive of electronic cigars, electronic cigarettes and the like.

When the word "about" is used in this specification in connection with a numerical value, it is intended that the associated numerical value include a tolerance of ±10% around the stated numerical value. Moreover, when reference is made to percentages in this specification, it is intended that those percentages are based on weight, i.e., weight percentages.

Moreover, when the words "generally" and "substantially" are used in connection with geometric shapes, it is intended that precision of the geometric shape is not required but that latitude for the shape is within the scope of the disclosure. When used with geometric terms, the words "generally" and "substantially" are intended to encompass not only features which meet the strict definitions but also features which fairly approximate the strict definitions.

It will now be apparent that a new, improved, and nonobvious electronic cigarette has been described in this specification with sufficient particularity as to be understood by one of ordinary skill in the art. Moreover, it will be apparent to those skilled in the art that numerous modifications, variations, substitutions, and equivalents exist for features of the electronic cigarette.

## Claims

1. An electronic cigarette comprising:
an outer cylindrical housing (22) extending in a longitudinal direction;
a central liquid supply (14,14a);
a capillary tube (18) having an inlet and an outlet, the inlet of the capillary tube in communication with an outlet of the central liquid supply (14,14a);
a heater (19) operable to heat the capillary tube to a temperature sufficient to at least initially volatilize a liquid material contained within the capillary tube,
the electronic cigarette **characterized by**:
the central liquid supply being formed of an elastomeric material and containing the liquid material, the liquid supply being arranged and adapted to be manually compressed so as to pump the liquid material from the liquid supply and through an outlet (16) of the liquid supply.

2. The electronic cigarette of Claim 1, wherein the liquid supply is at least partially contained within the outer cylindrical housing.

3. The electronic cigarette of Claim 1, further comprising:
a power supply (12) operable to apply voltage across the heater, the heater being adapted to be activated when the liquid supply is compressed;
a mixing chamber (46) downstream of the capillary tube; and
at least one air inlet (24) operable to deliver air drawn into the mixing chamber, the air being mixed with the volatilized liquid material in the mixing chamber to form a vapor.

4. The electronic cigarette of Claim 1, wherein the capillary tube has an internal diameter of about 0.05 to 0.4 mm, and a length that is one of about 5 mm to about 72 mm and about 10 mm to 60 mm.

5. The electronic cigarette of Claim 1, wherein the capillary tube includes one of a stainless steel tube and a non-metallic tube.

6. The electronic cigarette of Claim 3, wherein the power supply includes a battery.

7. The electronic cigarette of Claim 6, wherein the heater is connected to the battery by two spaced apart electrical leads (26).

8. The electronic cigarette of Claim 3, further including control circuitry (11) operable to control a supply of power from the power supply to the heater.

9. The electronic cigarette of Claim 3, wherein at least one air inlet is located near the outlet of the capillary tube.

10. The electronic cigarette of Claim 8, wherein the control circuitry further includes a heater activation light (27) at an end of the electronic cigarette, the heater activation light operable to light up when the heater is activated.

11. The electronic cigarette of Claim 1, wherein the liquid supply includes a check valve (40) operable to maintain the liquid within the liquid supply, wherein the check valve opens in response to the manual compression of the liquid supply.

12. The electronic cigarette of Claim 8, further comprising:
a pressure switch (44, 44') adjacent the liquid supply, wherein the manual compression applied to the liquid supply simultaneously pumps the liquid into the capillary tube and applies pressure to the pressure switch to send a signal to the control circuitry to supply power to the heater.

13. The electronic cigarette of Claim 8, further comprising:
a pressure switch adjacent the liquid supply, wherein the pressure switch is located on an outer wall of the outer cylindrical housing, wherein the manual compression is applied to the pressure switch to simultaneously activate the heater and also apply pressure to the liquid supply to release the liquid from the liquid supply.

14. The electronic cigarette of Claim 8, wherein the electronic cigarette includes a first section (70), a second section (72) and a third section (73), wherein the first section contains the capillary tube, the second section contains the power supply and control circuitry and the third section contains the liquid supply.

15. The electronic cigarette of Claim 14, wherein the first section is reusable and the second section is replaceable.

16. The electronic cigarette of Claim 14, wherein the third section includes a fitting operable to secure the liquid supply within the electronic cigarette, wherein the fitting includes a recess (36) beneath the liquid supply operable to hold the pressure switch.

17. The electronic cigarette of Claim 1, wherein the outer cylindrical housing has a cutout (100) therein superposed with a wall of the liquid supply, the cutout operable to allow a smoker to access and manually compress the liquid supply.

18. The electronic cigarette of Claim 1, wherein the outer cylindrical housing includes a depression (102) superposed with a wall of the liquid supply, the depression being operable to indicate to a smoker where to apply the manual compression to pump the liquid from the liquid supply.

19. The electronic cigarette of Claim 3, further comprising:
a critical orifice (41) operative to limit said communication of liquid to the capillary tube to a maximum flow rate.

20. The electronic cigarette of claim 1, further comprising:
a pressure switch (44/44') operable to communicate electrical power from a power source (12) to the heater, the pressure switch adapted to be manually operated when the liquid supply is manually compressed,
wherein the capillary tube discharges the liquid communicated to the capillary tube in an at least partially volatized condition.

21. The electronic cigarette of Claim 20, further comprising:
a check valve (40) operable to maintain the liquid within the liquid supply during periods when the liquid supply is not manually compressed, the check valve (40) operable to discharge the liquid to the capillary tube when the liquid supply is compressed.

22. The electronic cigarette of Claim 20, wherein the pressure switch is located on an outer wall of the electronic cigarette.

23. A method, comprising:
activating a pressure switch (44,44') to communicate electrical power from a power source (12) to an electric heater (19) operative upon a capillary tube (18), wherein the capillary tube (18) discharges a liquid material communicated to the capillary tube (18) in an at least partially volatized condition;
discharging the at least partially volatized liquid material into a chamber (46) near an end of the capillary tube (18) to produce a vapor;
drawing the vapor from the chamber (46);
the method being **characterized by**:
delivering the liquid material to the capillary tube (18) by manually communicating pressure to a compressible central liquid supply (14,14a) containing the liquid material in communication with the capillary tube (18).

24. The method of Claim 23, wherein the manually communicating pressure to a compressible liquid supply also communicates pressure to the pressure switch.

25. The method of Claim 23, wherein the delivering the liquid step includes limiting liquid communication to pressures above a minimum pressure.

26. The method of Claim 23, wherein the delivering the liquid step further includes limiting liquid communication to a maximum flow rate.

27. The method of Claim 23, wherein the producing vapor further includes admitting air and mixing the admitted air with the discharged liquid.

28. The method of Claim 23, wherein the activating the pressure switch further includes sensing flow from the liquid supply.

29. The method of Claim 23, wherein the activating the pressure switch further includes sensing flow from the liquid supply with sensing a puff.

## Patentansprüche

1. Elektronische Zigarette umfassend:
eine äußere zylindrische Aufnahme (22), welche sich in einer Längsrichtung erstreckt;
eine mittige Flüssigkeitszufuhr (14, 14a);
ein Kapillarrohr (18), welches einen Einlass und einen Auslass aufweist, wobei der Einlass des Kapillarrohrs in Verbindung mit dem Auslass der Flüssigkeitszufuhr (14, 14a) ist; und
eine Heizvorrichtung (19), welche dazu bedient werden kann, das Kapillarrohr bis zu einer Temperatur zu heizen, welche ausreichend ist, um mindestens das innerhalb des Kapillarrohrs enthaltene flüssige Material anfänglich zu verflüchtigen,
wobei die elektronische Zigarette durch Folgendes gekennzeichnet ist:
die mittige Flüssigkeitszufuhr, welche aus einem elastomerischen Material gebildet ist und ein flüssiges Material enthält, wobei die Flüssigkeitszufuhr dazu angeordnet und angepasst ist, manuell zusammengedrückt zu werden, um das flüssige Material von der Flüssigkeitszufuhr aus und durch einen Auslass (16) der Flüssigkeitszufuhr zu pumpen.

2. Elektronische Zigarette nach Anspruch 1, wobei die Flüssigkeitszufuhr mindestens teilweise innerhalb der äußeren zylindrischen Aufnahme enthalten ist.

3. Elektronische Zigarette nach Anspruch 1, zusätzlich umfassend:
eine Stromversorgung (12), welche dazu bedient werden kann, eine Spannung durch die Heizvorrichtung anzulegen, wobei die Heizvorrichtung dazu angepasst ist, aktiviert zu werden, wenn die Flüssigkeitszufuhr zusammengedrückt wird;
eine Mischkammer (46) stromabwärts des Kapillarrohrs; und
mindestens einen Lufteinlass (24), welcher dazu bedient werden kann, in die Mischkammer gepumpte Luft zu liefern, wobei die Luft mit dem verflüchtigten flüssigen Material in der Mischkammer gemischt wird, um Dampf zu bilden.

4. Elektronische Zigarette nach Anspruch 1, wobei das Kapillarrohr einen Innendurchmesser von etwa 0,05 bis 0,4 mm, und eine Länge aufweist, welche aus von etwa 5 mm bis etwa 72 mm und von etwa 10 mm bis 60 mm gewählt wird.

5. Elektronische Zigarette nach Anspruch 1, wobei das Kapillarrohr eins aus einem Edelstahlrohr und einem nicht metallischen Rohr beinhaltet.

6. Elektronische Zigarette nach Anspruch 3, wobei die Stromversorgung eine Batterie beinhaltet.

7. Elektronische Zigarette nach Anspruch 6, wobei die Heizvorrichtung mittels zweier getrennten elektrischen Leitungen (26) mit der Batterie verbunden ist.

8. Elektronische Zigarette nach Anspruch 3, zusätzlich beinhaltend einen Steuerschaltkreis (11), welcher dazu bedient werden kann, die Versorgung von Strom von der Stromversorgung aus bis zu der Heizvorrichtung zu steuern.

9. Elektronische Zigarette nach Anspruch 3, wobei sich der mindestens eine Lufteinlass in der Nähe des Auslasses des Kapillarrohrs befindet.

10. Elektronische Zigarette nach Anspruch 8, wobei der Steuerschaltkreis zusätzlich eine Heizvorrichtungsaktivierungslicht (27) an einem Ende der elektronischen Zigarette beinhaltet, wobei das Heizvorrichtungsaktivierungslicht dazu bedient werden kann, aufzuleuchten, wenn die Heizvorrichtung aktiviert ist.

11. Elektronische Zigarette nach Anspruch 1, wobei die Flüssigkeitszufuhr ein Rückschlagventil (40) beinhaltet, welches dazu bedient werden kann, die Flüssigkeit innerhalb der Flüssigkeitszufuhr zu halten, wobei sich das Rückschlagventil als Reaktion auf das manuelle Zusammendrücken der Flüssigkeitszufuhr öffnet.

12. Elektronische Zigarette nach Anspruch 8, zusätzlich umfassend:
einen Druckschalter (44, 44'), welcher der Flüssigkeitszufuhr benachbart ist, wobei das manuelle Zusammendrücken, welches auf die Flüssigkeitszufuhr angelegt wird, gleichzeitig die Flüssigkeit in das Kapillarrohr pumpt und einen Druck auf den Druckschalter anlegt, um ein Signal an den Steuerschaltkreis zu senden, um die Heizvorrichtung mit Strom zu versorgen.

13. Elektronische Zigarette nach Anspruch 8, zusätzlich umfassend:
einen Druckschalter, welcher der Flüssigkeitszufuhr benachbart ist, wobei sich der Druckschalter auf einer Außenwand der äußeren zylindrischen Aufnahme befindet, wobei das manuelle Zusammendrücken auf den Druckschalter angelegt wird, um gleichzeitig die Heizvorrichtung zu aktivieren und auch Druck auf die Flüssigkeitszufuhr anzulegen, um Flüssigkeit aus der Flüssigkeitszufuhr freizugeben.

14. Elektronische Zigarette nach Anspruch 8, wobei die elektronische Zigarette einen ersten Abschnitt (70), einen zweiten Abschnitt (72) und einen dritten Abschnitt (73) beinhaltet, wobei der erste Abschnitt das Kapillarrohr enthält, der zweite Abschnitt die Stromversorgung und den Steuerschaltkreis enthält und der dritte Abschnitt die Flüssigkeitszufuhr enthält.

15. Elektronische Zigarette nach Anspruch 14, wobei der erste Abschnitt wiederverwendbar ist und der zweite Abschnitt austauschbar ist.

16. Elektronische Zigarette nach Anspruch 14, wobei der dritte Abschnitt eine Armatur beinhaltet, welche dazu bedient werden kann, die Flüssigkeitszufuhr innerhalb der elektronischen Zigarette zu befestigen, wobei die Armatur eine Ausnehmung (36) unterhalb der Flüssigkeitszufuhr beinhaltet, welche dazu bedient werden kann, den Druckschalter zu halten.

17. Elektronische Zigarette nach Anspruch 1, wobei die äußere zylindrische Aufnahme einen Ausschnitt (100) innerhalb derselben aufweist, welcher mit einer Wand der Flüssigkeitszufuhr überlagert ist, wobei der Ausschnitt dazu bedient werden kann, einem Raucher Zugang auf die Flüssigkeitszufuhr zu leisten und sie manuell zusammenzudrücken.

18. Elektronische Zigarette nach Anspruch 1, wobei die äußere zylindrische Aufnahme eine Vertiefung (102) beinhaltet, welche mit einer Wand der Flüssigkeitszufuhr überlagert ist, wobei die Vertiefung dazu bedient werden kann, einem Raucher hinzuweisen wo das manuelle Zusammendrücken anzulegen ist, um die Flüssigkeit von der Flüssigkeitszufuhr aus zu pumpen.

19. Elektronische Zigarette nach Anspruch 3, zusätzlich umfassend:
ein kritisches Loch (41), welches dazu betriebsfähig ist, die genannte Übertragung der Flüssigkeit auf das Kapillarrohr auf eine maximale Durchflussrate zu begrenzen.

20. Elektronische Zigarette nach Anspruch 1, zusätzlich umfassend:
einen Druckschalter (44, 44'), welcher dazu bedient werden kann, elektrischen Strom von einer Stromquelle (12) auf eine Heizvorrichtung zu übertragen, wobei der Druckschalter dazu angepasst ist, manuell betrieben zu werden, wenn die Flüssigkeitszufuhr manuell zusammengedrückt wird,
wobei das Kapillarrohr die dem Kapillarrohr übertragene Flüssigkeit in einem mindestens teilweise verflüchtigten Umstand abführt.

21. Elektronische Zigarette nach Anspruch 20, zusätzlich umfassend:
ein Rückschlagventil (40), welches dazu bedient werden kann, die Flüssigkeit innerhalb der Flüssigkeitszufuhr während Perioden zu halten, wenn die Flüssigkeitszufuhr nicht manuell zusammengedrückt wird, wobei das Rückschlagventil (40) dazu bedient werden kann, die Flüssigkeit zum Kapillarrohr abzuführen, wenn die Flüssigkeitszufuhr zusammengedrückt wird.

22. Elektronische Zigarette nach Anspruch 20, wobei sich der Druckschalter auf einer Außenwand der elektronischen Zigarette befindet.

23. Verfahren, umfassend:
das Aktivieren eines Druckschalters (44, 44'), um elektrischen Strom von einer Stromquelle (12) auf eine elektrische Heizvorrichtung (19), welche in Bezug auf ein Kapillarrohr (18) betriebsfähig ist, zu übertragen, wobei das Kapillarrohr (18) ein flüssiges Material, welches dem Kapillarrohr (18) in einem mindestens teilweise verflüchtigten Zustand übertragen wird, abführt;
das Abführen des mindestens teilweise verflüchtigten flüssigen Materials in eine Kammer (46), welche in der Nähe eines Endes des Kapillarrohrs (18) liegt, um Dampf zu erzeugen;
das Pumpen des Dampfes aus der Kammer (46);
wobei das Verfahren durch Folgendes gekennzeichnet ist:
das Liefern des flüssigen Materials zum Kapillarrohr (18) durch das manuelle Übertragen von Druck auf eine zusammendrückbare Flüssigkeitszufuhr (14, 14a), welche das flüssige Material in Verbindung mit dem Kapillarrohr (18) enthält.

24. Verfahren nach Anspruch 23, wobei das manuelle Übertragen von Druck auf eine zusammendrückbare Flüssigkeitszufuhr dem Druckschalter auch Druck überträgt.

25. Verfahren nach Anspruch 23, wobei der Schritt des Lieferns von Flüssigkeit das Begrenzen der Übertragung von Flüssigkeit auf Druckwerte oberhalb eines minimalen Druckes beinhaltet.

26. Verfahren nach Anspruch 23, wobei der Schritt des Lieferns von Flüssigkeit zusätzlich das Begrenzen der Übertragung von Flüssigkeit auf eine maximale Durchflussrate beinhaltet.

27. Verfahren nach Anspruch 23, wobei das Erzeugen von Dampf zusätzlich das Einlassen von Luft und das Mischen der eingelassenen Luft mit der abgeführten Flüssigkeit beinhaltet.

28. Verfahren nach Anspruch 23, wobei das Aktivieren des Druckschalters zusätzlich das Detektieren des Flusses aus der Flüssigkeitszufuhr beinhaltet.

29. Verfahren nach Anspruch 23, wobei das Aktivieren des Druckschalters zusätzlich das Detektieren des Flusses aus der Flüssigkeitszufuhr mit dem Detektieren eines Zuges beinhaltet.

## Revendications

1. Cigarette électronique comprenant :
un logement cylindrique extérieur (22) s'étendant dans une direction longitudinale ;
une alimentation de liquide central (14, 14a) ;
un tube capillaire (18) ayant une entrée et une sortie, l'entrée du tube capillaire étant en communication avec une sortie de l'alimentation de liquide central (14, 14a) ;
un réchauffeur (19) apte à réchauffer le tube capillaire à une température suffisante pour au moins initialement volatiliser une matière liquide contenue dans le tube capillaire,
la cigarette électronique étant **caractérisée en ce que** :
l'alimentation de liquide central est formé par une matière élastomère et contient la matière liquide, l'alimentation de liquide est aménagé et adapté pour être comprimé manuellement de manière à pomper la matière liquide à partir de l'alimentation de liquide et à travers une sortie (16) de l'alimentation de liquide.

2. Cigarette électronique selon la revendication 1, dans laquelle l'alimentation de liquide est au moins partiellement contenu dans le logement cylindrique extérieur.

3. Cigarette électronique selon la revendication 1, comprenant en outre :
une alimentation électrique (12) apte à appliquer une tension à travers le réchauffeur, le réchauffeur étant adapté pour être activé lorsque l'alimentation de liquide est comprimé ;
une chambre de mélange (46) en aval du tube capillaire, et
au moins une entrée d'air (24) apte à fournir de l'air introduit dans la chambre de mélange, l'air étant mélange avec la matière liquide volatilisée dans la chambre de mélange pour former une vapeur.

4. Cigarette électronique selon la revendication 1, dans laquelle le tube capillaire a un diamètre interne d'environ 0,05 à 0,4 mm et une longueur qui est une d'environ 5 mm à environ 72 mm et d'environ 10 mm à 60 mm.

5. Cigarette électronique selon la revendication 1, dans laquelle le tube capillaire comporte un d'un tube en acier inoxydable et un tube non métallique.

6. Cigarette électronique selon la revendication 3, dans laquelle l'alimentation électrique comporte une batterie.

7. Cigarette électronique selon la revendication 6, dans laquelle le réchauffeur est connecté à la batterie par deux conducteurs électriques séparés (26).

8. Cigarette électronique selon la revendication 3, comportant en outre une circuiterie de contrôle (11) apte à contrôler une alimentation d'énergie électrique de l'alimentation électrique au réchauffeur.

9. Cigarette électronique selon la revendication 3, dans laquelle au moins une entrée d'air est située près de la sortie du tube capillaire.

10. Cigarette électronique selon la revendication 8, dans laquelle la circuiterie de contrôle comporte en outre une lumière d'activation du réchauffeur (27) et une extrémité de la cigarette électronique, la lumière d'activation du réchauffeur étant apte à s'allumer lorsque le réchauffeur est activé.

11. Cigarette électronique selon la revendication 1, dans laquelle l'alimentation de liquide comporte un clapet anti-retour (40) apte à maintenir le liquide dans l'alimentation de liquide, dans laquelle le clapet anti-retour s'ouvre en réponse à la compression manuelle de l'alimentation de liquide.

12. Cigarette électronique selon la revendication 8, comprenant en outre :
un pressostat (44, 44') attenant à l'alimentation de liquide, dans laquelle la compression manuelle appliquée à l'alimentation de liquide pompe simultanément le liquide dans le tube capillaire et applique de la pression au pressostat pour envoyer un signal à la circuiterie de contrôle pour alimenter en énergie le réchauffeur.

13. Cigarette électronique selon la revendication 8, comprenant en outre :
un pressostat attenant à l'alimentation de liquide, dans laquelle le pressostat est situé sur une paroi extérieure du logement cylindrique extérieur, dans laquelle la compression manuelle est appliquée au pressostat pour simultanément activer le réchauffeur et appliquer également de la pression à l'alimentation de liquide pour libérer le liquide de l'alimentation de liquide.

14. Cigarette électronique selon la revendication 8, dans laquelle la cigarette électronique comporte une première section (70), une deuxième section (72) et une troisième section (73), dans laquelle la première section contient le tube capillaire, la deuxième section contient l'alimentation électrique et la circuiterie de contrôle et la troisième section contient l'alimentation de liquide.

15. Cigarette électronique selon la revendication 14, dans laquelle la première section est réutilisable et la deuxième section est remplaçable.

16. Cigarette électronique selon la revendication 14, dans laquelle la troisième section comporte une fixation apte à assurer l'alimentation de liquide dans la cigarette électronique, dans laquelle la fixation comporte un évidemment (36) sous l'alimentation de liquide apte à contenir le pressostat.

17. Cigarette électronique selon la revendication 1, dans laquelle le logement cylindrique extérieur possède une encoche (100) sur celui-ci superposée à une paroi de l'alimentation de liquide, l'encoche étant apte à permettre qu'un fumeur accède et comprime manuellement l'alimentation de liquide.

18. Cigarette électronique selon la revendication 1, dans laquelle le logement cylindrique extérieur comporte une dépression (102) superposée à une paroi de l'alimentation de liquide, la dépression étant apte à indiquer à un fumeur où appliquer la compression manuelle pour pomper le liquide à partir de l'alimentation de liquide.

19. Cigarette électronique selon la revendication 3, comprenant en outre :
un orifice à écoulement critique (41) opérationnel pour limiter ladite communication de liquide vers le tube capillaire à un débit maximal.

20. Cigarette électronique selon la revendication 1, comprenant en outre :
un pressostat (44/44') apte à communiquer l'énergie électrique de l'alimentation électrique (12) au réchauffeur, le pressostat étant adapte pour être commandé manuellement lorsque l'alimentation de liquide est comprimée manuellement,
dans laquelle le tube capillaire décharge le liquide communiqué vers le tube capillaire dans une condition au moins partiellement volatilisée.

21. Cigarette électronique selon la revendication 20, comprenant en outre :
un clapet anti-retour (40) apte à maintenir le liquide dans l'alimentation de liquide pendant des périodes lorsque l'alimentation de liquide n'est pas comprimée manuellement, le clapet anti-retour (40) étant apte à décharger le liquide dans le tube capillaire lorsque l'alimentation de liquide est comprimée.

22. Cigarette électronique selon la revendication 20, dans laquelle le pressostat est situé sur une paroi extérieure de la cigarette électronique.

23. Procédé comprenant :
l'activation d'un pressostat (44, 44') pour communiquer l'énergie électrique d'une alimentation électrique (12) à un réchauffeur électrique (19) opérationnel sur un tube capillaire (18), dans lequel le tube capillaire (18) décharge une matière liquide communiquée vers le tube capillaire (18) dans une condition au moins partiellement volatilisée ;
la décharge de la matière au moins partiellement volatilisée dans une chambre (46) près d'une extrémité du tube capillaire (18) pour produire une vapeur ;
l'extraction de la vapeur de la chambre (46) ;
la livraison de la matière liquide au tube capillaire (18) en communiquant manuellement de la pression à l'alimentation de liquide central compressible (14, 14a) contenant la matière liquide en communication avec le tube capillaire (18).

24. Procédé selon la revendication 23, dans lequel la communication manuelle de pression à une alimentation de liquide compressible communique également de la pression au pressostat.

25. Procédé selon la revendication 23, dans lequel l'étape de livraison de liquide comporte la limitation de la communication de liquide à des pressions au-dessus d'une pression minimale.

26. Procédé selon la revendication 23, dans lequel l'étape de livraison de liquide comporte en outre la limitation de la communication de liquide à un débit maximal.

27. Procédé selon la revendication 23, dans lequel la production de vapeur comporte en outre l'admission d'air et le mélange de l'air admis avec le liquide déchargé.

28. Procédé selon la revendication 23, dans lequel l'activation du pressostat comporte en outre la détection de l'écoulement de l'alimentation de liquide.

29. Procédé selon la revendication 23, dans lequel l'activation du pressostat comporte en outre la détection de l'écoulement de l'alimentation de liquide avec détection d'une bouffée.
